# EUROPÄISCHE PATENTSCHRIFT

(11) **EP 0 653 439 B1**
(45) Veröffentlichungstag und Bekanntmachung des Hinweises auf die Patenterteilung: **28.04.2004**
(21) Anmeldenummer: 94117513.5
(22) Anmeldetag: 07.11.1994
(51) Int. Cl.: C07H 21/00, A61K 31/70

(54) **Stabilisierte Oligonucleotide und deren Verwendung**
Stabilized oligonucleotids and the use thereof
Oligonucléotides stabilisés et leur utilisation

(30) Priorität: 12.11.1993 DE 4338704
(43) Veröffentlichungstag der Anmeldung: 17.05.1995
(60) Teilanmeldung: 01124078.5
(73) Patentinhaber: Aventis Pharma Deutschland GmbH, 65929 Frankfurt am Main (DE)
(72) Erfinder: Peyman, Anuschirwan, Dr., D-65779 Kelkheim (DE); Uhlmann, Eugen, Dr., D-61479 Glashütten (DE); Mag, Matthias, D-61440 Oberursel (DE); Kretzschmar, Gerhard, Dr., D-65760 Eschborn (DE); Helsberg, Matthias, Dr., D-65779 Kelkheim (DE); Winkler, Irvin, Dr., D-65835 Liederbach (DE)

(56) Entgegenhaltungen:
- WO-A-87/07300
- WO-A-88/07544
- WO-A-89/08146
- WO-A-92/03139
- WO-A-92/20348
- WO-A-92/20697
- WO-A-93/01286
- WO-A-93/17125
- WO-A-94/01551
- WO-A-94/02499
- WO-A-94/26888
- WO-A-95/01363
- P.N.A.S., 1993 Seiten 3860-4, J. LISZIEWICZ ET AL. 'Long-term treatment of human immunodeficiency virus-infected cells with antisense oligonucleotide phosphorothioates'
- CHEMICAL ABSTRACTS, Bd. 114, Nr. 7, 18 Februar 1991, Columbus, Ohio, US; Zusammenfassung Nr. 55778N, I. MAGRATH: 'Tumor-specific antisense oligonucleotides for controlling cancer' & US-A-7 450 252 (US DEPT HEALTH & HUMAN SERVICE)
- DATABASE WPI Derwent Publications Ltd., London, GB; AN 93-085860 & US-A-7 821 415 (US DEPT HEALTH & HUMAN SERVICE)
- DATABASE WPI Derwent Publications Ltd., London, GB; AN 92-230579 & JP-A-04 154 794 (YODOGAWA PHARM. CO.) , 1992
- ANTI-CANCER DRUG DESIGN, Bd. 8, 1993 Seiten 15-32, M.K. GHOSH ET AL. 'Phosphorothioate-phosphodiester oligonucleotide copolymers'
- NUCLEIC ACIDS RES., Bd. 17, 1989 Seiten 9193-9204, P.J. FURDON ET AL. 'RNase H cleavage of RNA hybridized to oligonucleotides containing methylphosphonate, phosphorothioate and phosphodiester bonds'

## Beschreibung

Die Erfindung betrifft neue stabilisierte Oligonucleotide, bei denen mindestens ein nichtendständiges Pyrimidin-Nucleosid modifiziert ist sowie deren Verwendung als Diagnostikum oder Arzneimittel zur Behandlung von Vireninfektionen, Krebs oder Erkrankungen, bei denen Integrine oder Zell-Zell-Adhäsionsrezeptoren wirken.

Antisense Oligonucleotide (AO) und Tripel Helix Bildende Oligonucleotide (TFO) erwiesen sich als spezifische Inhibitoren der Genexpression in einer Vielzahl von Systemen, sowohl in vitro als auch in vivo [Uhlmann & Peyman, Chem. Rev. 1990, 90, 543; Milligan et al., J. Med. Chem. 1993, 36, 1923; Stein & Cheng, Science 1993, 261, 1004].

Eines der Hauptprobleme bei der Verwendung natürlich vorkommender Phosphodiester (PO) Oligonucleotide ist deren rascher Abbau durch verschiedene nucleolytische Aktivitäten sowohl in Zellen, wie auch im Zellkultur-Medium. Zur Stabilisierung von Oligonucleotiden wurden verschiedene chemische Modifikationen eingesetzt. Eine Übersicht über den Stand der Technik geben beispielsweise Milligan et al., supra und Uhlmann & Peyman, supra. Die Stabilisierung gegen nucleolytischen Abbau kann durch die Modifikation oder Ersatz der Phosphatbrücke, des Zuckerbausteins, der Nucleobase oder durch Ersatz des Zucker-Phosphat-Rückgrats der Oligonucleotide erfolgen. Da die Phosphatbrücke das Zentrum des nucleolytischen Angriffs ist, wurden insbesondere zahlreiche Modifikationen der Internucleosidbrücke beschrieben. Die am häufigsten verwandten nucleaseresistenten Internucleosidbrücken sind Phosphorothioat- (PS), Methylphosphonat- (MeP) und Phosphorodithioat- (PA) Brücken.

Dabei ist zu beachten, daß mit Einführung von Modifikationen nicht nur die Stabilität gegen Nucleasen verändert wird, sondern gleichzeitig eine Vielzahl von Eigenschaften der Antisense Oligonucleotide bzw. Tripel-Helix bildenden Oligonucleotide, wie beispielsweise deren Zellgängigkeit, RNase H Aktivierung, Spezifität und die Fähigkeit zur Hybridisierung mit RNA (für AO) bzw. DNA (für TFO) etc.. Darüberhinaus gibt es Hinweise, daß die Serum-Stabilität, die oft als Kriterium für Nucleasestabilität herangezogen wird, nicht immer die intrazelluläre Aktivität wiederspiegelt [P. D. Cook in "Antisense Research and Applications", Crooke and Lebleu, Eds., CRC Press, Boca Raton, 1993, Chapter 9, S.149ff]. Neben der Nucleaseresistenz gibt daher die biologische Wirkung von Antisense Oligonucleotiden oder Tripel Helix bildenden Oligonucleotiden einen Aufschluß über die Güte solcher Modifikationen.

In der Frage, an welchen Positionen im Oligonucleotid solche Modifikationen idealerweise erfolgen sollten, sind folgende Strategien entwickelt worden [P. D. Cook (supra); Uhlmann & Peyman (supra); Milligan et al, (supra)]:

### I) Der Austausch aller Internucleosid-Brücken, beispielsweise zu all-PS-Oligonucleotiden.

Dieser Austausch führt zu äußerst nukleasestabilen Oligonucleotiden. Beispielsweise ist der Abbau durch Endonucleasen (S1-Nuclease) und durch die Endo/Exo-Nuclease P1 bei einem all-PS Oligonucleotid um den Faktor 2-45 gegenüber einem PO Oligonucleotid verlangsamt [Stein et al., Nucl. Acids Res. 1988, 16, 1763]. All-PS Oligonucleotide sind auch in intakten Zellen resistent. In Xenopus Oocyten oder Embrionen werden microinjizierte PO Oligonucleotide mit einer Halbwertszeit von dreißig Minuten abgebaut, während all-PS Oligonucleotide unter gleichen Bedingungen eine Halbwertszeit von mehr als drei Stunden aufweisen [Woolf et al., Nucl. Acids Res. 1990, 18, 1763]. Auch all-MeP Oligonucleotide sind äußerst nucleaseresistent.

Der Nachteil von all-PS bzw. all-MeP Oligonucleotiden gegenüber den PO Oligonucleotiden ist ihre verminderte Fähigkeit, mit der Target RNA stabile Hybride einzugehen. Ein weiterer Nachteil der all-PS Oligonucleotide sind die unspezifischen ("Nicht-antisense"-)Effekte, die mit dieser Verbindungsklasse oft beobachtet werden [Milligan et al., supra; Stein & Cheng, supra].

Auch andere uniform modifizierte Derivate, beispielsweise all-2'-O-Methyl-Derivate oder all α-2'-Desoxyribo-Derivate sind im allgemeinen gekennzeichnet durch den Verlust der Fähigkeit zur Aktivierung von RNase H.

### II) Copolymere modifizierter und unmodifizierter Phosphordiester-Brücken

Ghosh et al. [Anti-Cancer Drug Design 1993, 8, 15] beschreiben ein Phosphorothioat-Phosphordiester Oligonucleotid mit verschiedenen Prozentanteilen an PS-Brücken. Diese sind beispielsweise nach dem Schema [PS-PO-PO-PO]ₙ, [PO-[PS-PO]ₙ, [PS-PO]ₙ, [(PO)₂-(PS)₂]ₙ, [PO-PS,PS]ₙ aufgebaut. Sie lehren, daß für eine selektive Inhibierung der Translation ein Gehalt von mindestens 50 % PS-Brücken notwendig ist und, daß darunter ein scharfer Abfall der Wirkung eintritt. Die vorliegende Erfindung zeigt, daß diese Ergebnisse nicht richtig sind und daß mit der richtigen Positionierung der Modifikationen ein Gehalt von weit weniger als 50 % PS-Bindungen für selektive Inhibierung ausreicht (s.u.). Weiter lehren Ghosh et al., daß mit den unter III beschriebenen Endcapping/Gap-Technik gute Resultate erzielt werden.

Der alternierende Austausch jeder zweiten Internucleosid-Brücke, beispielsweise gegen MeP-Brücken, (Furdan et al., Nucl. Acids Res. 1989, 17, 9193), erzielt gegenüber den uniform modifizierten MeP-Oligonucleotiden keinen Vorteil. So bewirken die alternierend MeP-modifizierten Oligonucleotide beispielsweise ebenfalls keine Aktivierung der RNase H.
Oligonucleotide mit alternierenden Phosphat-O-Ethyl- oder Phosphat-O-isopropyl-Estern und alternierende MeP-Oligonucleotide erwiesen sich im Vergleich auch weniger aktiv als all-MeP- oder all-PS-Oligonucleotide [Marcus-Secura et al., Nucl. Acids Res. 1987, 15, 5749].

### III) Der Austausch von eins, zwei oder drei Internucleosid-Brücken am 5'- bzw. am 3'-Ende der Oligonucleotide (End-Capping) sowie der Austausch von eins, zwei oder drei Internucleosid-Brücken am 5'- und am 3'-Ende der Oligonucleotide (gap-Technik).

Zur Effektivität des end-cappings existieren teilweise gegensätzliche Resultate. Insbesondere das 3'-end-capping durch PS-, PA, oder MeP Brücken wird als Schutz gegen Nucleasen beschrieben [P. D. Cook, supra, Milligan et al., supra].
Schutz durch 3'-Endcapping wurde auch durch verschiedene andere Modifikationen erreicht. 3'-3'-Endcapping wurde von verschiedenen Autoren als Schutz gegen nucleolytischen Abbau beschrieben [Shaw et al., Nucl. Acids Res. 1991, 19, 747; Seliger et al., Nucleoside & Nucleotides 1991, 10, 469]. Eine weitere Variante des 3'-Endcappings ist die Einführung von Konjugat-Molekülen am 3'-Ende, was ebenfalls die Nucleasestabilität erhöht, wie beispielsweise 3'-Dodecanol oder 3'-Acridin [P.D. Cook, supra], oder 3-Amino-2,3-propandiol [WO92/20697].
Die gap-Technik, also der Austausch von eins, zwei oder drei Internucleosid-Brücken am 5'- und am 3'-Ende der Oligonucleotide, hat sich besonders bewährt, da, abgesehen von PS-Oligonucleotiden, die meisten uniformen Modifikationen mit einem Verlust der Fähigkeit zur RNase H Aktivierung und damit einem starken Aktivitätsverlust einhergehen. Auch hier wurden die verschiedensten Derivate, modifizierte Phosphodiester-Brücken, modifizierte Zucker, modifizierte Basen, wie beispielsweise MeP-, PS-, PA-, 2'-O-Alkyl-, 2'-F-derivatisierte Oligonucleotide zur Stabilisierung eingesetzt. Eine Zusammenfassung dieser Ergebnisse findet sich in P. D. Cook supra. Im "gap" genügt dann eine Sequenz von zwei bis vier PO-Bindungen, um die RNase H zu aktivieren.

Giles et al. [Anti-Cancer Drug Design 1993, 8, 33] beschreiben Methylphosphonat-Phosphodiester-Chimere Oligonucleotide, in denen das Gap an unmodifizierten PO-Brücken kontinuierlich von acht auf zwei Brücken verkürzt wurde. Dabei wurde ein Trend zur verbesserten Zellaufnahme bei verkürztem gap festgestellt, jedoch wurden die Oligonucleotide nicht auf ihre Antisense-Wirkung untersucht.

Einen interessanten Vergleich verschiedener Strategien liefert Hoke et al. [Nucl. Acids Res. 1991, 19, 5743]. Sie vergleichen die Wirkung von verschiedenen PSmodifizierten Antisense Oligonucleotiden gegen HSV-1 in Zell-Kultur. Ihre Ergebnisse bestätigen, daß 3'- bzw. 3' + 5'- endcapping Oligonucleotide (jeweils die ersten drei Internucleosidbrücken werden modifiziert) im Serum, vergleichbar den all-PS Oligonucleotiden, ausreichend gegen Nuclease-Abbau geschützt werden. Dagegen werden intern modifizierte (3 PS Brücken) und nur 5'-endcapping Oligonucleotide (die ersten drei Internucleosidbrücken werden ebenfalls modifiziert) rasch abgebaut. Dagegen fanden sie, daß für die Wirkung in der Zelle weder ein 5'noch ein 3'-endcapping oder beides ausreicht und schließen daraus, daß eine uniforme Modifikation (all-PS) nötig ist, um ausreichende Stabilität gegen Nucleasen in Zellen zu erreichen.

Es wurde nun überraschenderweise gefunden, daß Pyrimidin-Nucleoside in den Oligonucleotiden Schwachpunkte gegen Nuclease-Resistenz darstellen. Werden diese Stellen nun durch Modifikationen, welche die Nuclease-Resistenz erhöhen, geschützt, so führte dies wiederum zu einer erheblichen Stabilitäts- und Wirkungsverbesserung.

Gegenstand der Erfindung sind daher Oligonucleotide der Formel wobei mindestens ein nichtendständiges Pyrimidin-Nucleosid modifiziert ist und zusätzlich die 5' und / oder die 3' Enden des Oligonukleotids modifiziert sind.

Bevorzugt sind hierbei Oligonucleotide, bei denen 2 - 10, insbesondere 3 - 6 nichtendständige Pyrimidin-Nucleoside modifiziert sind, wobei vor allem nicht mehr als 8 aufeinanderfolgende Nucleotide modifiziert sein sollen.
Insbesondere sind Oligonucleotide bevorzugt, bei denen zusätzlich die 5'- und/oder 3'-Enden modifiziert sind, insbesondere solche, bei denen die ersten 1 - 5, insbesondere 1 - 3, vor allem 2 - 3 Nucleotide am 5'- und/oder 3'-Ende, vorzugsweise durch Phosphorothioatbrücken, Phosphorodithioatbrücken und/oder Methylphosphonatbrücken verbunden sind. Vor allem sind solche modifizierten Oligonucleotide bevorzugt, die ein oder mehrere Gruppen von mindestens 1 - 4, insbesondere 3 - 4, untereinander verbundene und nichtmodifizierte Nucleotide enthalten.
Beispielsweise zeigt Tabelle 1 das am 5'und 3'-Ende zweifach mit PS gecappte Antisense Oligonucleotid 01 gegen HSV-1, das bei einer Konzentration von 27 µM wirksam ist. Die Einführung von drei PS-Brücken an 5'- und 3'-Ende bringt eine Aktivitätssteigerung auf 9 ìM, der gleiche Effekt wird durch Einführung einer einzelnen weiteren PS-Brücke 3' zu einem Cytosinrest C (Antisense Oligonucleotid Nr. 03) erreicht. Die Einführung von zwei PS-Brücken 5'- bzw. 3'- zu T und C (Antisense Oligonucleotid Nr. 05) bzw. die Einführung von vier PS-Brücken 5'- bzw. 3'- zu T und C (Antisense Oligonucleotid Nr. 06) führt zu weiteren Aktivitätssteigerungen von MHK-Werten (Minimale Hemmkonzentration) von 3 bzw. 1 µM. Bei 1 µM liegt auch der MHK-Wert des entsprechenden all-PS Derivats. Durch Schutz der Pyrimidin-Nucleoside ließ sich also eine Stabilitäts- und AktivitätsErhöhung, vergleichbar dem all-modifizierten Oligonucleotid erreichen, ohne jedoch die oben beschriebenen Nachteile einer solch drastischen Änderung in Kauf nehmen zu müssen.

Die Stabilisierungen an den Pyrimidin-Positionen wie auch an den 5'- und/oder 3'-Enden können unabhängig voneinander folgendermaßen erfolgen:
a) Ersatz der 3'- und/oder der 5'-Phosphorsäurediesterbrücke, beispielsweise durch eine Phosphorothioat-, Phosphorodithioat-, NR¹R²-Phosphoramidat-, Boranophosphat-, Phosphat-(C₁-C₂₁)-O-Alkylester, Phosphat-[(C₆-C₁₂)Aryl-(C₁-C₂₁)-O-Alkyl]ester, 2,2,2-Trichlorodimethylethylphosphonat-, (C₁-C₈)Alkylphosphonat-, (C₆-C₁₂)-Arylphosphonat-Brücke.
   Bevorzugt ist der Ersatz durch eine Phosphorothioat-, Phosphorodithioat-,NR¹R²-Phosphoramidat-, Phosphat-O-Methylester-, Phosphat-O-ethylester-, Phosphat-Oisopropylester-, Methylphosphonat-, Phenylphosphonat-Brücke.
   Besonders bevorzugt ist der Ersatz durch eine Phosphorothioat-, Phosphorodithioat, Methylphosphonat-Brücke.
   Ganz besonders bevorzugt ist der Ersatz durch eine Phosphorothioat-Brücke.
   R¹ und R² stehen unabhängig voneinander für Wasserstoff oder für C₁-C₁₈-Alkyl, C₆-C₂₀-Aryl, (C₆-C₁₄)-Aryl-(C₁-C₈)-alkyl, -(CH₂)_{c}-[NH(CH₂)_{c}]_{d}-NR³R³ steht, worin c eine ganze Zahl von 2 bis 6 und d eine ganze Zahl von 0 bis 6 ist, und R³ unabhängig voneinander Wasserstoff, oder C₁-C₆-Alkyl oder C₁-C₄-Alkoxy-C₁-C₆alkyl ist; bevorzugt steht R¹ und R² für Wasserstoff, C₁-C₈-Alkyl oder Methoxyethyl, besonders bevorzugt für Wasserstoff, C₁-C₄-Alkyl oder Methoxyethyl. R¹ und R² können auch zusammen mit dem sie tragenden Stickstoffatom einen 5-6-gliedrigen heterocyclischen Ring bilden, der zusätzlich ein weiteres Heteroatom aus der Reihe O, S, N enthalten kann.
b) Ersatz der 3'- oder 5'-Phosphorsäurediesterbrücke durch "Dephospho"-Brücken [s. z.B. Uhlmann und Peyrnan in "Methods in Molecular Biology", Vol. 20: Protocols for Oligonucleotides and Analogs", S. Agrawal, Ed., Humana Press, Totowa 1993, Chapter 16, 355ff], beispielsweise durch Formacetal, 3'-Thioformacetal, Methylhydroxylamin, Oxim, Methylendimethylhydrazo, Dimethylensulfon, Silylgruppen.
   Bevorzugt ist der Ersatz durch Formacetale und 3'-Thioformacetale.
c) Ersatz des Zuckerphosphat-Rückgrats, beispielsweise durch "Morpholinonucleosid"-Oligomere [E. P. Stirchak et al., Nucleic Acids Res. 17 (1989) 6129].
d) Ersatz der β-D-2'-Desoxyribose, beispielsweise durch α-D-2'-Desoxyribose, L-2'-Desoxyribose, 2'-F-2'-Desoxyribose, 2'-O-(C₁-C₆)Alkyl-Ribose, 2'-O-(C₂-C₆)Alkenyl-Ribose, 2'-NH₂-2'-desoxyribose, β-D-Xylofuranose, α-Arabinofuranose, 2,4-Dideoxy-β-D-erythro-hexo-pyranose, und carbocyclische [z.B. Froehler, J.Am.Chem.Soc. 1992, 114, 8320] und offenkettige Zuckeranaloga [z.B. Vandendriessche et al., Tetrahedron 1993, 49, 7223], Bicyclo-Zuckeranaloga [z.B. M. Tarkov et al., Helv. Chim. Acta 1993, 76, 481].
   Bevorzugt ist der Ersatz durch 2'-F-2'-Desoxyribose, 2'-O-(C₁-C₆)Alkyl-Ribose, 2'-O-(C₂-C₆)Alkenyl-Ribose, 2'-NH₂-2'-desoxyribose.
   Besonders bevorzugt ist der Ersatz durch 2'-F-2'-Desoxyribose, 2'-O-(C₁-C₄)Alkyl-Ribose, 2'-O-(C₂-C₄)Alkenyl-Ribose, 2'-NH₂-2'-desoxyribose.
   Ganz besonders bevorzugt ist der Ersatz durch 2'-O-Methyl-, 2'-O-Allyl-, 2'-O-Butylribose,
e) Ersatz der natürlichen Nucleosid-Basen, beispielsweise durch 5-(Hydroxymethyl)uracil, 5-Aminouracil, Pseudouracil, Dihydrouracil, 5-(C₁-C₆)-Alkyluracil, 5-(C₂-C₆)-Alkenyl-uracil, 5-(C₂-C₆)-Alkinyl-uracil, 5-(C₁-C₆)-Alkyl-cytosin, 5-(C₂-C₆)-Alkenyl-cytosin, 5-(C₂-C₆)-Alkinyl-cytosin, 5-Fluoruracil, 5-Fluorcytosin, 5-Chloruracil, 5-Chlorcytosin, 5-Bromuracil, 5-Bromcytosin.
   Bevorzugt ist der Ersatz durch 5-(C₁-C₆)-Alkyl-uracil, 5-(C₂-C₆)-Alkenyl-uracil, 5-(C₂-C₆)-Alkinyl-uracil, 5-(C₁-C₆)-Alkyl-cytosin, 5-(C₂-C₆)-Alkenyl-cytosin, 5-(C₂-C₆)-Alkinyl-cytosin, 5-Fluoruracil, 5-Fluorcytosin, 5-Chloruracil, 5-Chlorcytosin, 5-Bromuracil, 5-Bromcytosin.
   Besonders bevorzugt ist der Ersatz durch 5-(C₃-C₆)-Alkyl-uracil, 5-(C₂-C₆)-Alkenyluracil, 5-(C₂-C₆)-Alkinyl-uracil, 5-(C₁-C₆)-Alkyl-cytosin, 5-(C₂-C₆)-Alkenyl-cytosin, 5-(C₂-C₆)-Alkinyl-cytosin.
   Ganz besonders bevorzugt ist der Ersatz durch 5-Pentinylcytosin, 5-Hexinyluracil, 5-Hexinylcytosin.

Von den obengenannten Modifikationen sind vor allem die Modifikationen aus den Gruppen a), b), c) und d), vor allem aus den Gruppen a) und d), insbesondere aus der Gruppe a) bevorzugt.

Zusätzlich können die erfindungsgemäßen Oligonucleotide beispielsweise am 3'und/oder 5'-Ende mit Molekülen verbunden (konjugiert) sein, welche die Eigenschaften von Antisense-Oligonucleotiden oder von Tripelhelix bildenden Oligonucleotiden (wie beispielsweise Zellpenetration, Nucleaseabbau, Affinität zur Target-RNA/DNA, Pharmakokinetik) günstig beeinflußen. Beispiele sind Konjugate mit Poly-Lysin, mit Interkalatoren wie Pyren, Acridin, Phenazin, Phenanthridin, mit fluoreszierenden Verbindungen wie Fluorescein, mit Cross-Linkern wie Psoralen, Azidoproflavin, mit lipophilen Molekülen wie C₁₂-C₂₀-Alkyl, oder deren Derivate, wie z.B. Hexamethylentetraamin, mit Terpenen, wie Farnesol oder Phytol, mit Lipiden wie 1,2-Di-hexadecyl-rac-glycerin, mit Steroiden wie Gallensäure, Cholesterin oder Testosteron, mit Vitaminen wie Vitamin E, mit Poly- bzw. Oligoethylenglycol, mit (C₁₂-C₁₈)-Alkyl-Phosphatdiestern, mit -O-CH₂-CH(OH)-O-(C₁₂-C₁₈)-Alkyl. Bevorzugt sind Konjugate mit lipophilen Molekülen wie C₁₂-C₂₀-Alkyl, mit Steroiden wie Cholesterin oder Testosteron, mit Poly- oder Oligoethylenglykol, mit Vitamin E, mit Interkalatoren wie Pyren, mit (C₁₄-C₁₈)-Alkyl-Phosphatdiestern, mit
-O-CH₂-CH(OH)-O-(C₁₂-C₁₆)-Alkyl.

Die Darstellung solcher Oligonucleotid-Konjugate ist dem Fachmann bekannt (s. z.B. Uhlmann & Peyman, Chem. Rev. 1990, 90, 543; M. Manoharan in Antisense Research and Applications", Crooke and Lebleu, Eds., CRC Press, Boca Raton, 1993, Chapter 17, S.303ff, EP 0552766A2).

Weiterhin können die erfindungsgemäßen Oligonucleotide am 3' und/oder am 5'-Ende 3'-3'- und 5'-5'-Inversionen [beschrieben beispielsweise in M. Koga et al., J. Org. Chem. 56 (1991) 3757] tragen.

Gegenstand der Erfindung sind weiterhin Verfahren zur Herstellung der erfindungsgemäßen Verbindungen nach dem Fachmann bekannten Verfahren, insbesondere die chemische Synthese, die Verwendung der erfindungsgemäßen Verbindungen zur Herstellung eines Arzneimittels sowie ein Verfahren zur Herstellung eines Arzneimittels, das dadurch gekennzeichnet ist, daß man die erfindungsgemäßen Oligonucleotide mit einem physiologisch annehmbaren Träger sowie gegebenenfalls geeigneten Zusatz- und/oder Hilfsstoffen vermischt.

Ganz generell erstreckt sich die vorliegende Erfindung auch auf die Verwendung der erfindungsgemäßen, therapeutisch wirksamen Oligonucleotiden zur Herstellung eines Arzneimittels. Als therapeutisch wirksame Oligonucleotide versteht man im allgemeinen Antisense Oligonucleotide, Tripelhelix-bildende-Oligonucleotide, Aptamere (RNA oder DNA-Moleküle die an spezifische Zielmoleküle, z. B. Proteine oder Rezeptoren, binden können (z.B. L.C. Bock et al., Nature 1992, 355, 564) oder Ribozyme (katalytische RNA, s. z.B. Castanetto et al., Critical Rev. Eukar. Gene Expr. 1992, 2, 331), insbesondere Antisense-Oligonucleotide.

Darüberhinaus ist ein weiterer Gegenstand der vorliegenden Erfindung die Verwendung der erfindungsmäßigen Oligonucleotide als Diagnostikum, beispielsweise zur Detektion der Präsenz oder Absenz oder der Menge eines spezifischen doppelsträngigen oder einzelsträngigen Nucleinsäuremoleküls in einer biologischen Probe.

Die Oligonucleotide haben für die erfindungsgemäße Verwendung eine Länge von ca. 6 - 100, vorzugsweise von ca. 10 - 40, insbesondere von ca. 12 - 25 Nucleotiden. Ansonsten gelten auch hier die oben beschriebenen Vorzugsbereiche, Modifikationen bzw. Konjugationen.

Die Arzneimittel der vorliegenden Erfindung können beispielsweise zur Behandlung von Erkrankungen, die durch Viren hervorgerufen werden, beispielsweise durch HIV, HSV-1, HSV-2, Influenza, VSV, Hepatitis B oder Papilloma Viren, verwendet werden.

Antisense Oligonucleotide, die gegen solche Targets wirksam sind, sind beispielsweise:
a) gegen HIV, z. B.
b) gegen HSV-1, z.B.
   Die Arzneimittel der vorliegenden Erfindung eignen sich beispielsweise auch zur Behandlung von Krebs. Beispielsweise können dabei Oligonucleotid-Sequenzen zum Einsatz kommen, die gegen Targets gerichtet sind, die für Krebsentstehung bzw. Krebswachstum verantwortlich sind. Solche Targets sind beispielsweise:
   1) Nucleare Onkoproteine wie beispielsweise c-myc, N-myc, c-myb, c-fos, c-fos/jun, PCNA, p120
   2) Cytoplasmische/Membran-assoziierte Onkoproteine wie beispielsweise EJ-ras, c-Ha-ras, N-ras, rrg, bcl-2, cdc-2, c-raf-1, c-mos, c-src, c-abl
   3) Zelluläre Rezeptoren wie beispielsweise EGF-Rezeptor, c-erbA, Retinoid-Rezeptoren, Protein-Kinase regulatorische Untereinheit, c-fms
   4) Cytokine, Wachstumsfaktoren, Extrazelluläre Matrix wie beispielsweise CSF-1, IL-6, IL-1a, IL-1 b, IL-2, IL-4, bFGF, Myeloblastin, Fibronectin,

   Erfindungsgemäße Antisense-Oligonucleotide, die gegen solche Targets wirksam sind, sind beispielsweise
a) gegen c-Ha-ras , z. B.
c) c-myc, z.B.
d) c-myb, z.B.
e) c-fos, z.B.
f) p120, z.B.
g) EGF-Rezeptor, z.B.
h) p53 Tumorsuppressor, z.B.
j) Antisense-Oligonucleotid gegen cdc2-Kinase:
k) Antisense-Oligonucleotid gegen PCNA (proliferating cell nuclear antigen):
I) Antisense-Oligonucleotid gegen IGF-1:
m) Antisense-Oligonucleotid gegen bFGF Translation Start Site:
n) Antisense-Oligonucleotid gegen bFGF Codon 58 ff
o) Antisense-Oligonucleotid gegen FGF-Receptor:
   Die Arzneimittel der vorliegenden Erfindung eignen sich beispielsweise ferner zur Behandlung von Erkrankungen, die durch Integrine oder Zell-Zell-Adhäsionsrezeptoren beeinflußt werden, beispielsweise durch VLA-4, VLA-2, ICAM oder ELAM.

Erfindungsgemäße Antisense-Oligonucleotide, die gegen solche Targets wirksam sind, sind beispielsweise
a) VLA-4, z.B.
b) ICAM, z.B.
c) ELAM-1, z. B.

Die Arzneimittel können z.B. in Form von pharmazeutischen Präparaten, die man oral, z.B. in Form von Tabletten, Dragees, Hart- oder Weichgelatinekapseln, Lösungen, Emulsionen oder Suspensionen verabreichen kann, verwendet werden. Sie können auch rektal z.B. in Form von Suppositorien oder parenteral z.B. in Form von Injektionslösungen verabreicht werden. Für die Herstellung von pharmazeutischen Präparaten können diese Verbindungen in therapeutisch inerten organischen und anorganischen Trägern verarbeitet werden. Beispiele von solchen Trägern für Tabletten, Dragees und Hartgelatinekapseln sind Laktose, Maisstärke oder Derivate davon, Talg und Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole. Die pharmazeutischen Präparate können auch Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten.
Eine bevorzugte Form der Verabreichung ist die Injektion. Hierzu werden die Antisense-Oligonucleotide in einer flüssigen Lösung, vorzugsweise in einem physiologisch annehmbaren Puffer, wie z.B. Hank's Lösung oder Ringer's Lösung, formuliert. Die Antisense-Oligonucleotide können aber auch in fester Form formuliert werden und vor dem Gebrauch gelöst oder suspendiert werden. Die für die systematische Verabreichung bevorzugten Dosierungen betragen ca. 0,01 mg/kg bis ca. 50 mg/kg Körpergewicht und Tag.

Die folgenden Beispiele sollen nun die Erfindung näher erläutern:

### Beispiel 1

### Oligonucleotidsynthese

Unmodifizierte Oligonucleotide wurden auf einem automatischen DNA Synthesizer (Applied Biosystems Model 380B oder 394) unter Anwendung der Standard Phosphoramidit-Chemie und Oxidation mit Jod synthetisiert. Zur Einführung von Phosphorthioat-Brücken in gemischten Phosphorothioaten und Phosphodiester Oligonucleotiden wurde anstelle von Jod mit TETD (Tetraethylthiuramdisulfid) oxidiert (Applied Biosystems User Bulletin 65). Nach Abspaltung vom festen Träger (CPG oder Tentagel) und Entfernung der Schutzgruppen mit konz. NH₃ bei 55°C während 18h, wurden die Oligonucleotide zunächst durch Butanol-Fällung (Sawadogo, Van Dyke, Nucl. Acids Res. 19 (1991) 674 ) gereinigt. Anschließend wurde das Natriumsalz erhalten durch Ausfällung aus einer 0.5 M NaCI Lösung mit 2.5 Volumenteilen Ethanol.

Die Einführung des [4-(1-pyrenyl)butanyl]phosphodiesters am 5'-Ende erfolgt wie in J. S. Mann et al. Bioconj. Chem. 3 (1992) 554 beschrieben.

Die Analyse der Oligonucleotide erfolgte durch
a) Analytische Gelelektrophorese in 20% Acrylamid, 8M Harnstoff und/oder
b) HPLC-Analyse: Waters GenPak FAX, Gradient CH₃CN (400ml), H₂O (1.6l), NaH₂PO₄ (3.1 g), NaCl (11.7g), pH6.8 (0.1M an NaCl) nach CH₃CN (400ml), H₂O (1.6l), NaH₂PO₄ (3.1g), NaCl (175.3g), pH6.8 (1.5M an NaCl) und/oder
c) Kapillargelelektrophorese Beckmann Kapillare eCAP™, U100P Gel Column, 65 cm length, 100 mm I.D., window 15 cm from one end, Puffer 140 µM Tris, 360mM Borsäure, 7M Harnstoff und/oder
d) Elektrospray Massenspektroskopie

Die Analytise der Oligonucleotide ergab, daß diese jeweils in einer Reinheit von größer 90% vorlagen.

Die Strukturen der synthetisierten Oligonucleotide sind in Tabelle 1 dargestellt.

### Beispiel 2

### Untersuchung der antiviralen Aktivität von Prüfsubstanzen gegen Herpesviren in vitro

Die antivirale Aktivität der Prüfsubstanzen gegen verschiedene humanpathogene Herpesviren wird im Zellkulturtestsystem untersucht.

Für den Versuch werden Affennierenzellen (Vero, 2x10⁵/ml) in serumhaltigem Dulbecco's MEM (5 % Fötales Kälberserum FCS) in 96-Napf-Mikrotiterplatten ausgesät und 24 h bei 37°C und 5 % CO₂ inkubiert. Das serumhaltige Medium wird dann abgesaugt und die Zellen werden zweimal mit serumfreiem Dulbecco's MEM (-FCS) überspült.

Die Testsubstanzen werden in H₂O auf eine Konzentration von 600 µM vorverdünnt und bei -18°C aufbewahrt. Für den Test erfolgen weitere Verdünnungsschritte in Dulbecco's Minimal Essential Medium (MEM). Je 100 µl der einzelnen Prüfsubstanzverdünnungen werden zusammen mit 100 µl serumfreiem Dulbecco's MEM (-FCS) zu den gespülten Zellen gegeben.

Nach 3 h Inkubation bei 37°C und 5 % CO₂ werden die Zellen mit Herpes simplex Virus Typ 1 (ATCC VR733, HSV-1 F-strain) oder mit Herpes simplex Virus Typ 2 (ATCC VR734, HSV-2 G-Strain) infiziert in Konzentrationen, bei denen der Zellrasen innerhalb von 3 Tagen vollkommen zerstört wird. Bei HSV-1 beträgt die Infektionsstärke 500 Plaque-bildende Einheiten (PFU) pro Napf, bei HSV-2 350 PFU/Napf. Die Versuchsansätze enthalten dann Prüfsubstanz in Konzentrationen von 80 µM bis 0,04 µM in MEM, ergänzt durch 100 U/ml Penicillin G und 100 mg/l Streptomycin. Alle Versuche werden als Doppelbestimmung durchgeführt mit Ausnahme der Kontrollen, die achtfach je Platte durchgeführt werden.

Die Versuchsansätze werden 17 h bei 37°C und 5 % CO₂ inkubiert. Die Cytotoxizität der Prüfsubstanzen wird nach 20 h Gesamtinkubationszeit durch mikroskopische Begutachtung der Zellkulturen bestimmt. Als Dosis tolerata maxima (DTM) wird die höchste Präparatkonzentration bezeichnet, die unter den genannten Versuchsbedingungen noch keine mikroskopisch erkennbaren Zellschädigungen hervorruft.

Es erfolgt daraufhin die Zugabe von FCS auf eine Endkonzentration von 4 % mit weiterer Inkubation für 55 h bei 37°C und 5 % CO₂. Die unbehandelten Infektionskontrollen zeigen dann einen kompletten cytopathischen Effekt (CPE). Nach mikroskopischer Begutachtung der Zellkulturen werden diese dann mit Neutralrot entsprechend dem Vitalfärbungsverfahren nach Finter (1966) angefärbt. Die antivirale Aktivität einer Testsubstanz wird definiert als minimale Hemmkonzentration (MHK), die benötigt wird, um 30-60 % der Zellen vor dem virusbedingten cytopathogenen Effekt zu schützen.

Tabelle 1: Aktivität von verschieden modifizierten Antisense-Oligonucleotiden gegen HSV-1 in Zellkultur. Die durch eine Phosphorothioatbrücke (P=S) ersetzte Phosphodiesterbindungen wurden in der Sequenz mit * markiert;

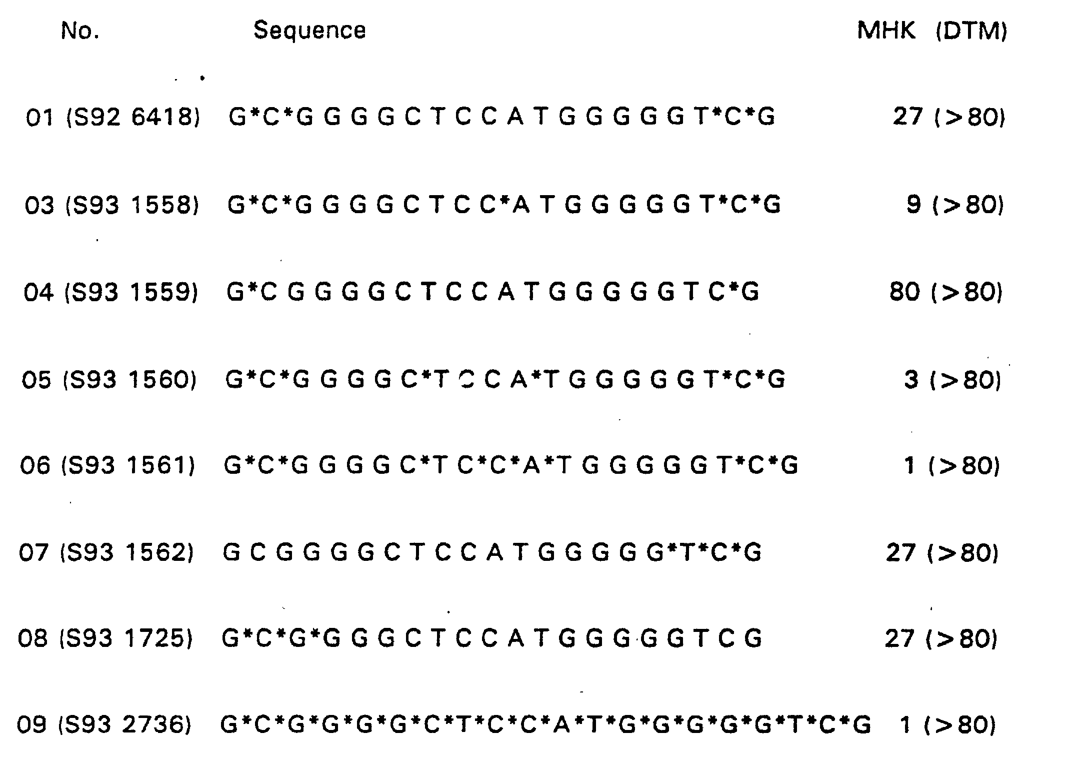

### Beispiel 3

### Untersuchung der Antiproliferationsaktivität von Prüfsubstanzen bei glatten Zellmuskeln

Die nachfolgend aufgeführten Oligonucleotide wurden auf ihre Fähigkeit überprüft, die Proliferation glatter Zellmuskeln zu inhibieren. Der Test wurde durchgeführt wie bei S. Biro et al. [Proc. Natl. Acad. Sci. USA 90 (1993) 654] beschrieben. Alle Oligonucleotide waren im Bereich 5 bis 20 µM wirksam. Die durch eine Phosphorothioatbrücke (P = S) ersetzte Phosphodiesterbindungen wurden in der Sequenz mit * markiert.
1) Antisense-Oligonucleotid gegen cdc2-Kinase:
2) Antisense-Oligonucleotid gegen PCNA (proliferating cell nuclear antigen):
3) Antisense-Oligonucleotid gegen IGF-1:
4) Antisense-Oligonucleotid gegen mouse c myb:
5) Antisense-Oligonucleotid gegen bFGF Translation Start site:
6) Antisense-Oligonucleotid gegen bFGF Codon 58 ff
7) Antisense-Oligonucleotid gegen FGF-Receptor:
8) Antisense-Oligonucleotid gegen c-myc:

### SEQUENZPROTOKOLL

### (1) ALLGEMEINE INFORMATION:

(i) ANMELDER:
   (A) NAME: Hoechst Aktiengesellschaft
   (B) STRASSE: -
   (C) ORT: Frankfurt am Main
   (D) BUNDESLAND: -
   (E) LAND: Deutschland
   (F) POSTLEITZAHL: 65926
   (G) TELEPHON: 069-305-6031
   (H) TELEFAX: 069-35 7175
   (I) TELEX: 41234700 hod
(ii) ANMELDETITEL: Stabilisierte Oligonucleotide und deren Verwendung
(iii) ANZAHL DER SEQUENZEN: 33
(iv) COMPUTER-LESBARE FORM:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0, Version #1.25 (EPA)

### (2) INFORMATION ZU SEQ ID NO: 1:

(i) SEQUENZ CHARAKTERISTIKA:
   (A) LÄNGE: 20 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: HIV
(ix) MERKMALE:
   (A) NAME/SCHLÜSSEL: exon
   (B) LAGE: 1..20
(xi) SEQUENZBESCHREIBUNG: SEQ ID NO: 1:
   ACACCCAATT CTGAAAATGG                                                    20

### (2) INFORMATION ZU SEQ ID NO: 2:

(i) SEQUENZ CHARAKTERISTIKA:
   (A) LÄNGE: 20 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: HIV
(ix) MERKMALE:
   (A) NAME/SCHLÜSSEL: exon
   (B) LAGE: 1..20
(xi) SEQUENZBESCHREIBUNG: SEQ ID NO: 2:
   AGGTCCCTGT TCGGGCGCCA 20

### (2) INFORMATION ZU SEQ ID NO: 3:

(i) SEQUENZ CHARAKTERISTIKA:
   (A) LÄNGE: 28 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: HIV
(ix) MERKMALE:
   (A) NAME/SCHLÜSSEL: exon
   (B) LAGE: 1..28
(xi) SEQUENZBESCHREIBUNG: SEQ ID NO: 3:
   GTCGACACCC AATTCTGAAA ATGGATAA 28

### (2) INFORMATION ZU SEQ ID NO: 4:

(i) SEQUENZ CHARAKTERISTIKA:
   (A) LÄNGE: 25 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: HIV
(ix) MERKMALE:
   (A) NAME/SCHLÜSSEL: exon
   (B) LAGE: 1..25
(xi) SEQUENZBESCHREIBUNG: SEQ ID NO: 4:
   GCTATGTCGA CACCCAATTC TGAAA 25

### (2) INFORMATION ZU SEQ ID NO: 5:

(i) SEQUENZ CHARAKTERISTIKA:
   (A) LÄNGE: 31 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: HIV
(ix) MERKMALE:
   (A) NAME/SCHLÜSSEL: exon
   (B) LAGE: 1..31
(xi) SEQUENZBESCHREIBUNG: SEQ ID NO: 5:
   TCGTCGCTGT CTCCGCTTCT TCTTCCTGCC A 31

### (2) INFORMATION ZU SEQ ID NO: 6:

(i) SEQUENZ CHARAKTERISTIKA:
   (A) LÄNGE: 31 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: HIV
(ix) MERKMALE:
   (A) NAME/SCHLÜSSEL: exon
   (B) LAGE: 1..31
(xi) SEQUENZBESCHREIBUNG: SEQ ID NO: 6:
   CTGTCTCCGC TTCTTCTTCC TGCCATAGGA G 31

### (2) INFORMATION ZU SEQ ID NO: 7:

(i) SEQUENZ CHARAKTERISTIKA:
   (A) LÄNGE: 20 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: HSV-1
(ix) MERKMALE:
   (A) NAME/SCHLÜSSEL: exon
   (B) LAGE: 1..20
(xi) SEQUENZBESCHREIBUNG: SEQ ID NO: 7:
   GCGGGGCTCC ATGGGGGTCG 20

### (2) INFORMATION ZU SEQ ID NO: 8:

(i) SEQUENZ CHARAKTERISTIKA:
   (A) LÄNGE: 15 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..15
   (D) SONSTIGE ANGABEN: /note= "c-Ha-ras"
(xi) SEQUENZBESCHREIBUNG: SEQ ID NO: 8:
   CAGCTGCAAC CCAGC 15

### (2) INFORMATION ZU SEQ ID NO: 9:

(i) SEQUENZ CHARAKTERISTIKA:
   (A) LÄNGE: 21 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..21
   (D) SONSTIGE ANGABEN: /note= "c-myc"
(xi) SEQUENZBESCHREIBUNG: SEQ ID NO: 9:
   GGCTGCTGGA GCGGGGCACA C                                              21

### (2) INFORMATION ZU SEQ ID NO: 10:

(i) SEQUENZ CHARAKTERISTIKA:
   (A) LÄNGE: 15 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..15
   (D) SONSTIGE ANGABEN: /note= "c-myc"
(xi) SEQUENZBESCHREIBUNG: SEQ ID NO: 10:
   AACGTTGAGG GGCAT                                                     15

### (2) INFORMATION ZU SEQ ID NO: 11:

(i) SEQUENZ CHARAKTERISTIKA:
   (A) LÄNGE: 15 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..15
   (D) SONSTIGE ANGABEN: /note= "c-myc"
(xi) SEQUENZBESCHREIBUNG: SEQ ID NO: 11:
   CACGTTGAGG GGCAT                                                       15

### (2) INFORMATION ZU SEQ ID NO: 12:

(i) SEQUENZ CHARAKTERISTIKA:
   (A) LÄNGE: 18 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..18
   (D) SONSTIGE ANGABEN: /note= "c-myb"
(xi) SEQUENZBESCHREIBUNG: SEQ ID NO: 12:
   GTGCCGGGGT CTTCGGGC                                                    18

### (2) INFORMATION ZU SEQ ID NO: 13:

(i) SEQUENZ CHARAKTERISTIKA:
   (A) LÄNGE: 18 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Maus
(ix) MERKMALE:
   (A) NAME/SCHLÜSSEL: exon
   (B) LAGE: 1..18
   (D) SONSTIGE ANGABEN: /note= "c-myb"
(xi) SEQUENZBESCHREIBUNG: SEQ ID NO: 13:
   GTGTCGGGGT CTCCGGGC 18

### (2) INFORMATION ZU SEQ ID NO: 14:

(i) SEQUENZ CHARAKTERISTIKA:
   (A) LÄNGE: 21 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..21
   (D) SONSTIGE ANGABEN: /note= "c-fos"
(xi) SEQUENZBESCHREIBUNG: SEQ ID NO: 14:
   GGAGAACATC ATGGTCGAAA G 21

### (2) INFORMATION ZU SEQ ID NO: 15:

(i) SEQUENZ CHARAKTERISTIKA:
   (A) LÄNGE: 22 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..22
   (D) SONSTIGE ANGABEN: /note= "c-fos"
(xi) SEQUENZBESCHREIBUNG: SEQ ID NO: 15:
   CCCGAGAACA TCATGGTCGA AG                                              22

### (2) INFORMATION ZU SEQ ID NO: 16:

(i) SEQUENZ CHARAKTERISTIKA:
   (A) LÄNGE: 20 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..20
   (D) SONSTIGE ANGABEN: /note= "c-fos"
(xi) SEQUENZBESCHREIBUNG: SEQ ID NO: 16:
   GGGGAAAGCC CGGCAAGGGG                                                 20

### (2) INFORMATION ZU SEQ ID NO: 17:

(i) SEQUENZ CHARAKTERISTIKA:
   (A) LÄNGE: 20 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..20
   (D) SONSTIGE ANGABEN: /note= "p-120"
(xi) SEQUENZBESCHREIBUNG: SEQ ID NO: 17:
   CACCCGCCTT GGCCTCCCAC                                              20

### (2) INFORMATION ZU SEQ ID NO: 18:

(i) SEQUENZ CHARAKTERISTIKA:
   (A) LÄNGE: 18 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..18
   (D) SONSTIGE ANGABEN: /note= "EGF-Rezeptor"
(xi) SEQUENZBESCHREIBUNG: SEQ ID NO: 18:
   GGGACTCCGG CGCAGCGC 18

### (2) INFORMATION ZU SEQ ID NO: 19:

(i) SEQUENZ CHARAKTERISTIKA:
   (A) LÄNGE: 20 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..20
   (D) SONSTIGE ANGABEN: /note= "EGF-Rezeptor"
(xi) SEQUENZBESCHREIBUNG: SEQ ID NO: 19:
   GGCAAACTTT CTTTTCCTCC 20

### (2) INFORMATION ZU SEQ ID NO: 20:

(i) SEQUENZ CHARAKTERISTIKA:
   (A) LÄNGE: 19 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..19
   (D) SONSTIGE ANGABEN: /note= "p53 Tumorsuppressor"
(xi) SEQUENZBESCHREIBUNG: SEQ ID NO: 20:
   GGGAAGGAGG AGGATGAGG 19

### (2) INFORMATION ZU SEQ ID NO: 21:

(i) SEQUENZ CHARAKTERISTIKA:
   (A) LÄNGE: 21 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..21
   (D) SONSTIGE ANGABEN: /note= "p53 Tumorsuppressor"
(xi) SEQUENZBESCHREIBUNG: SEQ ID NO: 21:
   GGCAGTCATC CAGCTTCGGA G 21

### (2) INFORMATION ZU SEQ ID NO: 22:

(i) SEQUENZ CHARAKTERISTIKA:
   (A) LÄNGE: 18 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..18
   (D) SONSTIGE ANGABEN: /note= "cdc2-Kinase"
(xi) SEQUENZBESCHREIBUNG: SEQ ID NO: 22:
   GTCTTCCATA GTTACTCA 18

### (2) INFORMATION ZU SEQ ID NO: 23:

(i) SEQUENZ CHARAKTERISTIKA:
   (A) LÄNGE: 18 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..18
   (D) SONSTIGE ANGABEN: /note= "PCNA (proliferating cell nuclear antigen)"
(xi) SEQUENZBESCHREIBUNG: SEQ ID NO: 23:
   GATCAGGCGT GCCTCAAA 18

### (2) INFORMATION ZU SEQ ID NO: 24:

(i) SEQUENZ CHARAKTERISTIKA:
   (A) LÄNGE: 21 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..21
   (D) SONSTIGE ANGABEN: /note= "IGF-1"
(xi) SEQUENZBESCHREIBUNG: SEQ ID NO: 24:
   TGAAGACGAC ATGATGATGT G                    21

### (2) INFORMATION ZU SEQ ID NO: 25:

(i) SEQUENZ CHARAKTERISTIKA:
   (A) LÄNGE: 15 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..15
   (D) SONSTIGE ANGABEN: /note= "bFGF Translation Start Site"
(xi) SEQUENZBESCHREIBUNG: SEQ ID NO: 25:
   GGCTGCCATG GTCCC                           15

### (2) INFORMATION ZU SEQ ID NO: 26:

(i) SEQUENZ CHARAKTERISTIKA:
   (A) LÄNGE: 19 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..19
   (D) SONSTIGE ANGABEN: /note= "bFGF Codon 58 ff"
(xi) SEQUENZBESCHREIBUNG: SEQ ID NO: 26:
   CTGTAGTTTG ACGTGTGGG 19

### (2) INFORMATION ZU SEQ ID NO: 27:

(i) SEQUENZ CHARAKTERISTIKA:
   (A) LÄNGE: 22 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..22
   (D) SONSTIGE ANGABEN: /note= "FGF-Receptor"
(xi) SEQUENZBESCHREIBUNG: SEQ ID NO: 27:
   GGCCCCTCCA GCCCCACATC CC 22

### (2) INFORMATION ZU SEQ ID NO: 28:

(i) SEQUENZ CHARAKTERISTIKA:
   (A) LÄNGE: 18 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..18
   (D) SONSTIGE ANGABEN: /note= "VLA-4"
(xi) SEQUENZBESCHREIBUNG: SEQ ID NO: 28:
   GCAGTAAGCA TCCATATC 18

### (2) INFORMATION ZU SEQ ID NO: 29:

(i) SEQUENZ CHARAKTERISTIKA:
   (A) LÄNGE: 20 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..20
   (D) SONSTIGE ANGABEN: /note= "ICAM"
(xi) SEQUENZBESCHREIBUNG: SEQ ID NO: 29:
   CCCCCACCAC TTCCCCTCTC 20

### (2) INFORMATION ZU SEQ ID NO: 30:

(i) SEQUENZ CHARAKTERISTIKA:
   (A) LÄNGE: 20 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..20
   (D) SONSTIGE ANGABEN: /note= "ICAM"
(xi) SEQUENZBESCHREIBUNG: SEQ ID NO: 30:
   CTCCCCCACC ACTTCCCCTC 20

### (2) INFORMATION ZU SEQ ID NO: 31:

(i) SEQUENZ CHARAKTERISTIKA:
   (A) LÄNGE: 19 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..19
   (D) SONSTIGE ANGABEN: /note= "ICAM"
(xi) SEQUENZBESCHREIBUNG: SEQ ID NO: 31:
   GCTGGGAGCC ATAGCGAGG 19

### (2) INFORMATION ZU SEQ ID NO: 32:

(i) SEQUENZ CHARAKTERISTIKA:
   (A) LÄNGE: 21 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..21
   (D) SONSTIGE ANGABEN: /note= "ELAM-1"
(xi) SEQUENZBESCHREIBUNG: SEQ ID NO: 32:
   ACTGCTGCCT CTTGTCTCAG G 21

### (2) INFORMATION ZU SEQ ID NO: 33:

(i) SEQUENZ CHARAKTERISTIKA:
   (A) LÄNGE: 22 Basenpaare
   (B) ART: Nukleinsäure
   (C) STRANGFORM: Einzel
   (D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
   (A) ORGANISMUS: Mensch
(ix) MERKMALE:
   (A) NAME/SCHLÜSSEL: exon
   (B) LAGE: 1..22
   (D) SONSTIGE ANGABEN: /note= "ELAM-1"
(xi) SEQUENZBESCHREIBUNG: SEQ ID NO: 33:
   CAATCAATGA CTTCAAGAGT TC 22

## Patentansprüche

1. Oligonucleotide der Formel **dadurch gekennzeichnet, daß** mindestens ein nicht-endständiges Pyrimidin-Nucleosid modifiziert ist und zusätzlich die 5' und/oder die 3'Enden des Oligonukleotids modifiziert sind.

2. Oligonucleotide nach Anspruch 1, **dadurch gekennzeichnet, daß** 2 bis 10 nicht-endständige Pyrimidin-Nucleoside modifiziert sind.

3. Oligonucleotide nach Anspruch 1 oder 2, **dadurch gekennzeichnet, daß** 3 bis 6 nicht-endständige Pyrimidin-Nucleoside modifiziert sind.

4. Oligonucleotide nach einem der Ansprüche 1 - 3, **dadurch gekennzeichnet, daß** ein oder mehrere Gruppen von mindestens 1 - 4 miteinander verbundenen Nukleotiden nicht modifiziert sind.

5. Oligonucleotide nach einem der Ansprüche 1 - 4, **dadurch gekennzeichnet, daß** die Modifikation definiert ist als
(a) Ersatz mindestens einer 3'- und/oder 5'-Phosphorsäurediesterbindung benachbarter Nucleotide und/oder
(b) Dephosphobindung benachbarter Nucleotide und/oder
(c) Ersatz mindestens eines Zuckerrestes und/oder
(d) Ersatz mindestens eines natürlich vorkommenden Nucleosides.

6. Oligonucleotide nach Anspruch 5, **dadurch gekennzeichnet, daß** die Modifikation definiert ist als
(a) eine Phosphorothioat-, Phosphorodithioat-, NR¹R²⁻Phosphoramidat-, Boranophosphat-, Phosphat-(C₁-C₂₁)-O-Alkylester, Phosphat-[(C₆-C₁₂)Aryl-(C₁-C₂₁)-O-Alkyl]ester, 2,2,2-Trichlorodimethylethylphosphonat-, (C₁-C₈)Alkylphosphonat-, (C₆-C₁₂)-Arylphosphonat-Bindung benachbarter Nucleotide darstellt, wobei
R¹ und R² unabhängig voneinander für Wasserstoff oder für C₁-C₁₈-Alkyl, C₆-C₂₀-Aryl, (C₆-C₁₄)-Aryl-(C₁-C₈)-alkyl, -(CH₂)_{c}-[NH(CH₂)_{c}]_{d}-NR³R³ steht, worin c eine ganze Zahl von 2 bis 6 und d eine ganze Zahl von 0 bis 6 ist, und R³ unabhängig voneinander Wasserstoff, oder C₁-C₆-Alkyl oder C₁-C₄-Alkoxy-C₁-C₆-alkyl ist, steht oder
R¹ und R² bilden zusammen mit dem sie tragenden Stickstoffatom einen 5-6-gliedrigen heterocyclischen Ring, der gegebenenfalls zusätzlich ein weiteres Heteroatom aus der Reihe O, S, N enthalten kann,
(b) eine Formacetal-, 3'-Thioformacetal-, Methylhydroxylamin-, Oxim-, Methylendimethylhydrazo-, Dimethylensulfon-, Silyl-Bindung benachbarter Nucleotide,
(c) Ersatz des Zuckerphosphat-Rückgrats durch "Morpholinonucleosid"-Oligomere oder
(d) Ersatz der β-D-2'-Desoxyribose, durch α-D-2'-Desoxyribose, L-2'-Desoxyribose, 2'-F-2'-Desoxyribose, 2'-O-(C₁-C₆)Alkyl-Ribose, 2'-O-(C₂-C₆)Alkenyl-Ribose, 2'-NH₂-2'-desoxyribose, β-D-Xylofuranose, α-Arabinofuranose, 2,4-Dideoxy-β-D-erythro-hexo-pyranose, carbocyclische oder offenkettige Zuckeranaloga oder Bicyclo-Zuckeranaloga,
(e) Ersatz der natürlichen Nucleosid-Basen durch 5-(Hydroxymethyl)uridin, 5-Aminouridin, Pseudouridin, Dihydrouridin, 5-(C₁-C₆)-Alkyl-uridin, 5-(C₁-C₆)-Alkenyl-uridin, 5-(C₁-C₆)-Alkinyl-uridin, 5-(C₁-C₆)-Alkyl-cytidin, 5-(C₁-C₆)-Alkenyl-cytidin, 5-(C₁-C₆)-Alkinyl-cytidin, 5-Fluoruridin, 5-Fluorcytidin, 5-Chloruridin, 5-Chlorcytidin, 5-Bromuridin oder 5-Bromcytidin.

7. Oligonucleotide nach einem der Ansprüche 1 - 6, **dadurch gekennzeichnet, daß** das Oligonucleotid am 5' und/oder am 3'-Ende einen lipophilen Rest, vorzugsweise einen Famesyl-, Phytyl-, Hexadecyl-, Cholesteryl-, Hexamethylentetraamin-, Vitamin E- oder Gallensäure-Rest trägt.

8. Verfahren zur Herstellung der Oligonucleotide nach einem der Ansprüche 1 - 7, **dadurch gekennzeichnet, daß** sie chemisch synthetisiert werden.

9. Verfahren zur Herstellung eines Arzneimittels, **dadurch gekennzeichnet, daß** mindestens ein Oligonucleotid nach einem der Ansprüche 1 - 7 mit einem physiologisch annehmbaren Träger sowie gegebenenfalls geeigneten Zusatzund/oder Hilfsstoffen vermischt wird.

10. Verwendung der Oligonucleotide nach einem der Ansprüche 1 - 7 zur Herstellung eines Arzneimittels oder Diagnostikums.

## Claims

1. An oligonucleotide of the formula wherein at least one non-terminal pyrimidine nucleoside is modified and the 5' and/or 3' ends of the oligonucleotide are additionally modified.

2. An oligonucleotide as claimed in claim 1, wherein 2-10 non-terminal pyrimidine nucleosides are modified.

3. An oligonucleotide as claimed in claim 1 or 2, wherein 3-6 non-terminal pyrimidine nucleosides are modified.

4. An oligonucleotide as claimed in any of claims 1-3, wherein one or more groups of at least 1-4 nucleotides which are linked to each other are not modified.

5. An oligonucleotide as claimed in any of claims 1-4, wherein the modification is defined as
(a) replacement of at least one 3' and/or 5' phosphodiester bond of adjacent nucleotides and/or
(b) dephospho bonding of adjacent nucleotides and/or
(c) replacement of at least one sugar radical and/or
(d) replacement of at least one naturally occurring nucleoside.

6. An oligonucleotide as claimed in claim 5, wherein the modification is defined as
(a) a phosphorothioate, phosphorodithioate, NR¹R²-phosphoramidate, boranophosphate, phosphate-(C₁-C₂₁)-O-alkyl ester, phosphate-[(C₆-C₁₂)aryl-(C₁-C₂₁)-O-alkyl] ester, 2,2,2-trichlorodimethylethyl phosphonate, (C₁-C₈)-alkyl phosphonate, (C₆-C₁₂)-aryl phosphonate bond of adjacent nucleotides, in which
R¹ and R² independently of one another are hydrogen or C₁-C₁₈-alkyl, C₆-C₂₀-aryl, (C₆-C₁₄)-aryl-(C₁-C₈)-alkyl or -(CH₂)_{c}-[NH(CH₂)_{c}]_{d}-NR³R³, in which c is an integer from 2 to 6 and d is an integer from 0 to 6, and R³ radicals independently of one another are hydrogen, C₁-C₆-alkyl or C₁-C₄-alkoxy-C₁-C₆-alkyl, or
R¹ and R² together with the nitrogen atom to which they are attached form a 5-6-membered heterocyclic ring which can optionally additionally contain a further heteroatom from the series consisting of O, S and N,
(b) a formacetal, 3'-thioformacetal, methylhydroxylamine, oxime, methylenedimethylhydrazo, dimethylene sulfone or silyl bond of adjacent nucleotides,
(c) a replacement of the sugar-phosphate backbone by morpholinonucleoside oligomers or
(d) a replacement of the β-D-2'-deoxyribose by β-D-2'-deoxyribose, L-2'-deoxyribose, 2'-F-2'-deoxyribose, 2'-O-(C₁-C₆)alkyl-ribose, 2'-O-(C₂-C₆)alkenyl-ribose, 2'-NH₂-2'-deoxyribose, β-D-xylofuranose, α-arabinofuranose, 2,4-dideoxy-β-D-erythro-hexo-pyranose, carbocyclic or open-chain sugar analogs or bicyclo sugar analogs, or
(e) a replacement of the natural nucleoside bases by 5-(hydroxymethyl)uridine, 5-aminouridine, pseudouridine, dihydrouridine, 5-(C₁-C₆)-alkyluridine, 5-(C₁-C₆)-alkenyluridine, 5-(C₁-C₆)-alkynyluridine, 5-(C₁-C₆)-alkylcytidine, 5-(C₁-C₆)-alkenylcytidine, 5-(C₁-C₆)-alkynylcytidine, 5-fluorouridine, 5-fluorocytidine, 5-chlorouridine, 5-chlorocytidine, 5-bromouridine or 5-bromocytidine.

7. An oligonucleotide as claimed in any of claims 1-6, wherein the oligonucleotide has a lipophilic radical, preferably a famesyl, phytyl, hexadecyl, cholesteryl, hexamethylenetetraamine, vitamin E or bile acid radical, on the 5' and/or the 3' end.

8. A process for the preparation of the oligonucleotide as claimed in any of claims 1-7, which comprises their chemical synthesis.

9. A process for the preparation of a pharmaceutical, which comprises mixing at least one oligonucleotide as claimed in any of claims 1-7 with a physiologically acceptable excipient and, if appropriate, suitable additives and/or auxiliaries.

10. The use of the oligonucleotide as claimed in any of claims 1-7 for the preparation of a pharmaceutical or diagnostic.

## Revendications

1. Oligonucléotides de formule **caractérisés en ce qu'**au moins un pyrimidine-nucléoside non terminal est modifié et de plus, les extrémités 5' et/ou 3' de l'oligonucléoside sont modifiées.

2. Oligonucléotides selon la revendication 1, **caractérisés en ce que** 2 à 10 pyrimidine-nucléosides non terminaux sont modifiés.

3. Oligonucléotides selon la revendication 1 ou 2, **caractérisés en ce que** 3 à 6 pyrimidine-nucléosides non terminaux sont modifiés.

4. Oligonucléotides selon l'une des revendications 1 à 3, **caractérisés en ce qu'**un ou plusieurs groupes d'au moins 1 à 4 nucléotides reliés entre eux ne sont pas modifiés.

5. Oligonucléotides selon l'une des revendications 1 à 4, **caractérisés en ce que** la modification est définie comme
(a) remplacement d'au moins une liaison phosphodiester 3' et/ou 5' des nucléotides adjacents et/ou
(b) liaison diphospho des nucléotides adjacents et/ou
(c) remplacement d'un reste glucidique et/ou
(d) remplacement d'au moins un nucléoside naturel.

6. Oligonucléotides selon la revendication 5, **caractérisés en ce que** la modification est définie comme
(a) une liaison phosphorothioate, phosphoridithioate, NR¹R²-phosphoramidate, boranophosphate, phosphate-O-(alkyl-en C₁-C₂₁)ester, phosphate-(aryl en C₆-C₁₂)-(alkyl-O-en C₁-C₂₁), une liaison 2,2,2-trichlorodiméthyléthylphosphonate, (alkyl en C₁-C₈)-phosphonate, (aryl en C₆-C₁₂)phosphonate des nucléotides adjacents,
R¹ et R² représentent, indépendamment l'un de l'autre, un atome d'hydrogène ou un groupe alkyle en C₁-C₁₈, aryle en C₆-C₂₀, (aryl en C₆-C₁₄)-(alkyle en C₁-C₈), -(CH₂)_{c}-[NH(CH₂)_{c}]_{d}-NR³R³, où c est un entier de 2 à 6 et d un entier de 0 à 6, et R³ représentent, indépendamment l'un de l'autre un atome d'hydrogène ou un groupe alkyle en C₁-C₆ ou (alkoxy en C₁-C₄)-(alkyle en C₁-C₆) ou
R¹ et R² forment conjointement avec l'atome d'azote qui les porte, un cycle hétérocyclique de 5 à 6 chaînons, qui peut éventuellement contenir en plus un autre hétéroatome pris parmi les atomes O, S, N,
(b) une liaison formacétal, 3'-thioformacétal, méthylhydroxylamine, oxime, méthylènediméthylhydrazo, diméthylènesulfone, silyle, des nucléotides adjacents,
(c) remplacement de la colonne sucre-phosphate par des oligomères "morpholinonucléoside" ou
(d) remplacement du β-D-2'-désoxyribose par l'α-D-2'-désoxyribose, le L-2'-désoxyribose, le 2'-F-2'-désoxyribose, le 2'-O-(alkyl en C₁-C₆)ribose, le 2'-O-(alcényl en C₂-C₆)-ribose, le 2'-NH₂-2'-désoxyribose, le β-D-xylofuranose, l'α-arabinofuranose, le 2,4-didésoxy-β-D-érythrohexopyranose, des analogues glucidiques carboxyliques ou à chaîne ouverte ou analogues bicyclo-glucidiques,
(e) remplacement des bases nucléosides naturelles par la 5-(hydroxyméthyl)uridine, la 5-aminouridine, la pseudouridine, la dihydrouridine, la 5-(alkyl en C₁-C₆)-uridine, la 5-(alcényl en C₁-C₆)uridine, la 5-(alcynyl en C₁-C₆)uridine, la 5-(alkyl en C₁-C₆)cytidine, la 5-(alcényl en C₁-C₆)cytidine, la 5-(alcynyl en C₁-C₆)-cytidine, la 5-fluoruridine, la 5-fluorcytidine, la 5-chloruridine, la 5-chlorocytidine, la 5-bromuridine ou la 5-bromocytidine.

7. Oligonucléotides selon l'une des revendications 1 à 6, **caractérisés en ce que** l'oligonucléotide porte à l'extrémité 5' et/ou 3' un reste lipophile, de préférence un reste farnésyle, phytyle, hexadécyle, cholestéryle, hexaméthylènetétraamine, vitamine E ou acide biliaire.

8. Procédé pour la préparation des oligonucléotides selon l'une des revendications 1 à 7, **caractérisé en ce qu'**ils sont synthétisés par voie chimique.

9. Procédé pour la préparation d'un médicament **caractérisé en ce qu'**on mélange au moins un oligonucléotide selon l'une des revendications 1 à 7 avec un véhicule physiologiquement acceptable ainsi qu'éventuellement avec des matières d'addition et/ou adjuvants.

10. Utilisation des oligonucléotides selon l'une des revendications 1 à 7 pour la préparation d'un médicament ou d'un produit diagnostique.
